(19) Europäisches Patentamt
European Patent Office
Office européen des brevets

(11) Publication number: **0 099 685 B1**

# (12) EUROPEAN PATENT SPECIFICATION

(45) Date of publication of patent specification: **21.09.88**

(21) Application number: **83303850.8**

(22) Date of filing: **01.07.83**

(51) Int. Cl.[4]: **C 07 D 213/30,** C 07 D 213/32, C 07 D 213/36, C 07 D 213/65, C 07 D 213/74, C 07 D 213/75

## (54) (3-Pyridinyl)heteroalkanols and alkanoic acids and esters.

(30) Priority: **16.07.82 US 399141**

(43) Date of publication of application:
**01.02.84 Bulletin 84/05**

(45) Publication of the grant of the patent:
**21.09.88 Bulletin 88/38**

(84) Designated Contracting States:
**BE CH DE FR GB IT LI NL SE**

(56) References cited:
**DE-A-2 637 911**
**GB-A-2 068 950**

**CHEMICAL ABSTRACTS vol. 68, no. 25, 17 June 1968, Columbus, Ohio, USA; M.P. MERTES et al. "Synthesis of 3-pyridoxyalkanoic acids and their derivatives", page 11019, column1, abstract no. 114371f & J. Heterocycl. Chem. vol. 5, no. 2, 1968, pages 281-283**

(73) Proprietor: **THE UPJOHN COMPANY**
**301 Henrietta Street**
**Kalamazoo, Michigan 49001 (US)**

(72) Inventor: **Lin, Chiu-Hong c/o The Upjohn Company**
**301 Henrietta Street**
**Kalamazoo Michigan 49001 (US)**

(74) Representative: **Perry, Robert Edward et al**
**GILL JENNINGS & EVERY 53-64 Chancery Lane**
**London WC2A 1HN (GB)**

**EP 0 099 685 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to ω-(3-pyridinyl)oxa, thia-, and aza- alkanoic acids and esters thereof. Such compounds are potent thromboxane $A_2$ inhibitors and as such represent useful pharmacological agents.

Since the discovery that human platelets convert the prostaglandin endoperoxide ($PGG_2$) into a labile proaggregatory molecule known as thromboxane $A_2$ ($TXA_2$), researchers have sought compounds that could selectively inhibit the biological activity of $TXA_2$. This end may be achieved in two different ways: the synthesis of $TXA_2$ can be blocked by inhibiting the $TXA_2$ synthetase, or a compound could be a receptor level antagonist of $TXA_2$. As therapeutic agents, $TXA_2$ synthetase inhibitors are more useful. See, e.g., R. Gorman, Biological and Pharmacological Evaluation of Thromboxane Synthetase Inhibitors, Advances in Prostaglandin and Thromboxane Research, 6:417 (1980), and references cited therein.

A number of thromboxane inhibitors are known. See for example the bi-heterocyclic 9,11-trideoxy-PGF-type compounds disclosed in US—A—4,112,224; SQ 80,388 (1-(3-phenyl-2-propenyl)-1H-imidazole) disclosed in D. Harris, et al., Advances in Prostaglandin and Thromboxane Research 6:437 (1980); pyridine and its derivatives, disclosed in T. Miyamoto, et al., Advances in Prostaglandin and Thromboxane Research, 6:443 (1980), and GB—A—2,039,903 (abstracted in Derwent Farmdoc No. 50111C (1980)); see also H. Tai, et al., Advances in Prostaglandin and Thromboxane Research, 6:447 (1980). Other compounds which have been disclosed as thromboxane synthetase inhibitors include sodium p-benzyl-4(1-oxo-2-(4-chlorobenzyl)-3-phenylpropyl)phenyl phosphate, imidazoles, nordihydroguaiaretic acid, and 12-hydroperoxy-5,8,10,14-eicosatetraenoic acid (HETE). As noted in the above British patent specification, however, the inhibitory activity of these latter compounds on thromboxane synthetase is very weak, making them unsatisfactory as practically effective medicines.

US—A—2,559,546 discloses certain 3-pyridoxy-alkanoic acids which are stated to be useful as bactericides. Certain alkyl-N-arylcarbamates are disclosed by Leardini *et al*, Synthesis 3:225 (1982). Kaimya, Chem. Pharm. Bull. 23:2744 (1975), discloses N-(3-pyridyl)methylurethane as an intermediate for certain carcinogenic compounds. Similar compounds, having longer alkyl side chains, are disclosed by Rouvier *et al*, Bull. Soc. Chim. Fr. 5:1717, 1718 (1971).

GB—A—2068950 discloses compounds analogous to formula I (see Chart A), but in which $NR_2$ is replaced by —O— or —S—, while m is 0 or 1, n + p = 1—6, $Y_1$ is a valence bond and $Q_1$ is, say, COOH. These compounds and analogues are stated to be useful as thromboxane synthetase inhibitors.

Compounds according to the present invention are of the formula I, wherein

$Y_1$ is —O—, —S—, —$NR_2$— or a valence bond;

$R_2$ is H, $C_{1-5}$ alkyl or CHO;

$Q_1$ is $CH_2OH$ or $COOR_1$ wherein $R_1$ is H, a pharmacologically-acceptable cation, $C_{1-12}$ alkyl, $C_{3-10}$ cycloalkyl, $C_{7-12}$ aralkyl, phenyl or phenyl substituted by one, 2 or 3 substituents independently selected from Cl and $C_{1-3}$ alkyl;

m is zero or an integer of 1 to 6;

n is an integer of 2 to 6; and

p is zero or an integer of 1 to 6;

provided that n + p is at least 3 if $Y_1$ is a valence bond.

"$C_{1-3}$ alkyl" means methyl, ethyl, propyl or isopropyl. "$C_{1-12}$ alkyl" means methyl, ethyl, propyl, butyl, pentyl, hexyl, heptyl, octyl, nonyl, decyl, undecyl, dodecyl, and isomeric forms thereof.

Examples of $C_{3-10}$ cycloalkyl, which includes alkyl-substituted cycloalkyl, are cyclopropyl, 2-methylcyclopropyl, 2,2-dimethylcyclopropyl, 2,3-diethylcyclopropyl, 2-butylcyclopropyl, cyclobutyl, 2-methylcyclobutyl, 3-propylcyclobutyl, 2,3,4-triethylcyclobutyl, cyclopentyl, 2,2-dimethylcyclopentyl, 2-pentylcyclopentyl, 3-tert-butylcyclopentyl, cyclohexyl, 4-tert-butylcyclohexyl, 3-isopropylcyclohexyl, 2,2-dimethylcyclohexyl, cycloheptyl, cyclooctyl, cyclononyl and cyclodecyl.

Examples of $C_{7-12}$ aralkyl are benzyl, 2-phenethyl, 1-phenylethyl, 2-phenylpropyl, 4-phenylbutyl, 3-phenylbutyl, 2-(1-naphthylethyl), and 1-(2-naphthylmethyl).

Examples of phenyl substituted by one to 3 chloro or alkyl of one to 3 carbon atoms, inclusive, are p-chlorophenyl, m-chlorophenyl, 2,4-dichlorophenyl, 2,4,6-trichlorophenyl, p-tolyl, m-tolyl, o-tolyl, p-ethylphenyl, 2,5-dimethylphenyl, 4-chloro-2-methylphenyl, and 2,4-dichloro-3-methylphenyl.

The compounds of the present invention may be in the form of pharmacologically acceptable salts. These salts are formed when $R_1$ is a pharmacologically acceptable cation. Such cations include: pharmacologically acceptable metal cations, ammonium, amine cations, or quaternary ammonium cations.

Especially preferred metal cations are those derived from the alkali metals, e.g., lithium, sodium, and potassium, and from the alkaline earth metals, e.g., magnesium and calcium, although cationic forms of other metals, e.g., aluminum, zinc, and iron are within the scope of this invention.

Pharmacologically acceptable amine cations are those derived from primary, secondary, or tertiary amines. Examples of suitable amines are methylamine, dimethylamine, trimethylamine, ethylamine, dibutylamine, triisopropylamine, N-methylhexylamine, decylamine, dodecylamine, allylamine, crotylamine, cyclopentylamine, dicyclohexylamine, benzylamine, dibenzylamine, α-phenylethylamine, β-phenylethylamine, ethylenediamine, diethylenetriamine, and aliphatic, cycloaliphatic, araliphatic amines containing up

to and including 18 carbon atoms, as well as heterocyclic amines, e.g., piperidine, morpholine, pyrrolidine, piperazine, and lower-alkyl derivatives thereof, e.g.

1-methylpiperidine,
4-ethylmorpholine,
1-isopropylpyrrolidine,
2-methylpyrrolidine,
1,4-dimethylpiperazine,
2-methylpiperidine,

as well as amines containing water-solubilizing or hydrophilic groups, e.g.,

mono-, di-, and triethanolamine,
ethyldiethanolamine,
N-butylethanolamine,
2-amino-1-butanol,
2-amino-2-ethyl-1,3-propanediol,
2-amino-2-methyl-1-propanol,
tris(hydroxymethyl)aminomethane,
N-phenylethanolamine,
N-(p-tert-amylphenyl)diethanolamine,
glactamine,
N-methylglycamine,
N-methylglucosamine,
ephedrine,
phenylephrine,
epinephrine,
procaine.

Further useful amine salts are the basic amino acid salts, e.g.

lysine and
arginine.

Examples of suitable pharmacologically acceptable quaternary ammonium cations are

tetramethylammonium,
tetraethylammonium,
benzyltrimethylammonium,
phenyltriethylammonium.

Pharmaceutically acceptable acid addition salts are formed at the pyridinyl moiety and are also useful for administering the compounds of this invention. These salts include hydrochloride, hydrobromide, hydroiodide, sulfate, phosphate, acetate, propionate, lactate, maleate, malate, succinate, tartrate. They are prepared by methods well known in the art.

The compounds of the present invention will be named herein using the Chemical Abstracts numbering system (see Naming and Indexing of Chemical Substances for Chemical Abstracts during the Ninth Collective Period (1972—1976), a reprint of section IV from the Volume 76 Index Guide).

The compounds of the present invention are tested for $TXA_2$ inhibitions as follows:

Rabbit aortic strips were superfused in series with Krebs solution. Thromboxane $A_2$ ($TXA_2$) was generated by mixing prostaglandin $H_2$ ($PGH_2$) with human platelet microsomes (HPM).

Potential inhibitors were tested by comparing the response of the rabbit aorta to the amount of $TXA_2$ produced by mixing $PGH_2$ and HPM without the test compound in the reaction medium and then the amount of $TXA_2$ produced when the test compound was added to the HPM 5 minutes before the HPM was mixed with $PGH_2$. By this means compounds which selectively inhibit $TXA_2$ synthetase are found. For a discussion of $TXA_2$ synthetase inhibition testing see, e.g., R. Gorman, *supra*.

Using this test system, sodium salt (Example 2), has been shown to be the most effective in inhibiting $TXA_2$ formation. This compound has an approximately $ED_{50}$ in this system of between 10 and 100 ng/ml.

The novel compounds of this invention have thus been shown to be highly active as inhibitors of the thromboxane synthetase enzyme system. Accordingly, these novel compounds are useful for administration to mammals, including humans, whenever it is desirable medically to inhibit this enzyme system. For a discussion of the utility of $TXA_2$ inhibitors, see, e.g., Derwent Farmdoc Nos. 18399B; 72896B; 72897B; 63409B; 03755C; 03768C; and 50111C.

Thus, for example, these novel compounds are useful as antiinflammatory agents in mammals and especially humans, and for this purpose, are administered systemically and preferably orally. For oral administration, a dose range of 0.05 to 50 mg per kg of human body weight is used to give relief from pain associated with inflammatory disorders such as rheumatoid arthritis. They are also administered intravenously in aggravated cases of inflammation, preferably in a dose range 0.01 to 100 µg per kg per minute until relief from pain is attained. When used for these purposes, these novel compounds cause fewer and lesser undesirable side effects than do the known synthetase inhibitors used to treat inflammation, for example, aspirin and indomethacin. When these novel compounds are administered orally, they are formulated as tablets, capsules, or as liquid preparations, with the usual pharmaceutical carriers and binders. For intravenous use, sterile isotonic solutions are preferred.

These compounds are useful whenever it is desired to inhibit platelet aggregation, reduce the adhesive character of platelets, and remove or prevent the formation of thrombi in mammals, including man, rabbits, and rats. For example, these compounds are useful in the treatment and prevention of myocardial infarcts, to treat and prevent post-operative thrombosis, to promote patency of vascular grafts following surgery, and to treat conditions such as atherosclerosis, arteriosclerosis, blood clotting defects due to lipemia, and other clinical conditions in which the underlying etiology is associated with lipid imbalance or hyperlipidemia. For these purposes, these compounds are administered systemically, e.g., intravenously, subcutaneously, intramuscularly, and in the form of sterile implants for prolonged action. For rapid response especially in emergency situations, the intravenous route of administration is preferred. Doses in the range about 0.005 to about 20 mg per kg of body weight per day are used, the exact dose depending on the age, weight, and condition of the patient or animal, and on the frequency and route of administration.

These compounds are further useful as additives to blood, blood products, blood substitutes, or other fluids which are used in artificial extracorporeal circulation or perfusion of isolated body portions, e.g., limbs and organs, whether attached to the original body, detached and being preserved or prepared for transplant, or attached to a new body. During these circulations and perfusions, aggregated platelets tend to block the blood vessels and portions of the circulation apparatus. This blocking is avoided by the presence of these compounds. For this purpose, the compound is added gradually or in single or multiple portions to the circulating blood, to the blood of the donor animal, to the perfused body portion, attached or detached, to the recipient, or to two or all of these at a total steady state dose of about 0.001 to 10 mg per liter of circulating fluid. It is especially useful to use these compounds in laboratory animals, e.g., cats, dogs, rabbits, monkeys, and rats, for these purposes in order to develop new methods and techniques for organ and limb transplants.

The compounds of the present invention are useful in mammals, including humans and certain useful animals, e.g., dogs and pigs, to reduce or avoid gastrointestinal ulcer formation, and accelerate the healing of such ulcers already present in the gastrointestinal tract. For this purpose, these compounds are injected or infused intravenously, subcutaneously, or intramuscularly in an infusion dose range about 0.1 μg to about 500 μg/kg of body weight per minute, or in a total daily dose by injection or infusion in the range about 0.1 to about 20 mg/kg of body weight per day, the exact dose depending on the age, weight, and condition of the patient or animal, and on the frequency and route of administration.

The novel compounds are used for the purposes described above in the free acid form, in ester form, and in the pharmacologically acceptable salt form. When the ester form is used, the ester is any of those within the above definition of $R_1$. However, it is preferred that the ester be alkyl of one to 12 carbon atoms, inclusive. Of the alkyl esters, methyl and ethyl are especially preferred for optimum absorption of the compound by the body or experimental animal system; and straight-chain octyl, nonyl, decyl, undecyl, and dodecyl are especially preferred for prolonged activity in the body or experimental animal.

Thromboxane synthetase converts $PGH_2$ (prostaglandin endoperoxide) into $TXA_2$. $PGH_2$ is also converted to prostacyclin, $PGD_2$, and other compounds by other enzymes. Thus, because the compounds of this invention inhibit thromboxane $A_2$ synthetase, they increase the $PGH_2$ substrate and thus increase the amount of endogenous prostacyclin. Therefore, they are also useful for many of the pharmacological purposes for which prostacyclin is employed.

Prostacyclin and a thromboxane synthetase inhibitor have both been shown to be effective in controlling tumor cell metastasis, see, e.g., K. Honn, et al., "Thromboxane Synthetase Inhibitors and Prostacyclin Can Control Tumor Cell Metastasis," an Abstract of the Twentieth Annual Meeting of the American Society for Cell Biology, in the Journal of Cell Biology, 87:64 (1980).

Similarly, prostacyclin has been shown to be an effective antihypertensive agent. The compounds of the present invention are also used for this purpose. (See, e.g., GB—A—2 039 903).

For a general discussion of the utility of $TXA_2$ synthetase inhibitors which increase endogenous prostacyclin, see, Aiken, et al., J. Pharmacol. Exp. Ther., 219:299 (1981).

The compounds of the present invention are prepared by the methods depicted in Chart B.

In Chart B, $R_{22}$ is $C_{1-12}$ alkyl, OTs represents a tosylate, and $R_{23}$ is an amine-protecting group such as formyl, acetyl, trifluoroacetyl or trifluoromethanesulfonyl (J. B. Hendrickson and R. Bergeron, Tetr. Lett., 3839 (1973)).

An amine of the formula B—1 is protected with an amine-protecting group by means well known in the art, e.g. a formyl group from formic acid and acetic anhydride, to give a compound of the formula B—2 wherein $R_{23}$ is —CHO. The amine of the formula B—2 is treated with a halide of the formula $Z_1$—$(CH_2)_{n-1}$—$COOR_{22}$ ($Z_1$ is Cl, Br or I) and sodium hydride in a solvent such as DMF to yield the formula B—3 compound of the present invention. Pharmacologically-acceptable salts are prepared by methods described above to yield, e.g., the formula B—5 compound wherein $R_2$ is —CHO and $R_1$ is sodium. Alternatively, the formula B—3 compound wherein $R_{23}$ is formyl may be hydrolyzed by known methods, e.g. treatment with hydrochloric acid in methanol-water, to give the formula B—4 compound wherein $R_2$ is hydrogen. Treatment of the formula B—4 compound with sodium hydroxide in methanol yields the formula B—5 compound wherein $R_2$ is hydrogen and $R_1$ is sodium. The formula B—3 compound may be reduced by known methods, e.g. lithium aluminium hydride, to yield the formula B—6 compound.

Repeated alkylation of the formula B—6 compound with a halide of the formula $Z_1$—$(CH_2)_p$—$COOR_{22}$ gives the compound of the formula B—7. Conversion of the formula B—6 compound with p-toluenesulfonyl chloride in pyridine, as is well known in the art, gives the tosylate of the formula B—10. Alkylation of the formula B—10 compound with the anion generated from the thiol derivative of the formula $HS—(CH_2)_p—COOR_{22}$ and base, e.g. methyl mercaptopropionate with sodium methoxide in methanol, yields the sulfide of the formula B—11 wherein $Y_1$ is —S—. Alkylation of the formula B—10 compound with the anion generated from the amino derivative of the formula $HNR_2—(CH_2)_p—COOR_{22}$ and base, as described above regarding the conversion of the formula B—2 compound to the formula B—3 compound, yields the compound of the formula B—11 wherein $Y_1$ is $—NR_2—$.

The formula B—7 or B—11 compounds are respectively converted to B—8 and then B—9, or B—12 and then B—13 compounds.

Certain compounds of the present invention are preferred. Thus, compounds of the formula I wherein the sum of m + n + p is 2 to 7, $R_2$ is H, $Y_1$ is a valence bond, $Q_1$ is $COOR_1$ and $R_1$ is hydrogen, methyl or a pharmacologically-acceptable cation are preferred. Also preferred are compounds of the formula I wherein the sum of m + n + p is 2 to 7, $R_2$ is CHO, $Y_1$ is —O— or a valence bond, $Q_1$ is $COOR_1$ and $R_1$ is hydrogen, methyl or a pharmacologically-acceptable cation.

The following Preparations and Examples illustrate how compounds of the present invention may be prepared.

## Example 1

6-[Formyl(3-pyridinyl)amino]hexanoic acid, methyl ester (Formula I: m is zero, $R_2$ is CHO, n + p = 5, $Y_1$ is a bond, and $Q_1$ is $CO_2CH_3$)

Refer to Chart B (conversion of B—1 to B—2 to B—3).

A two-neck flask equipped with a magnetic stirring bar and a dropping funnel is dried and flushed with nitrogen. A 50% emulsion of sodium hydride (6.0 mmol, 288 mg) is placed in the flask and washed twice with dry hexane. To this suspension, 470 mg (5.0 mmol) of 3-aminopyridine are added and the resulting deep green solution is stirred vigorously. The mixture is stirred until the evolution of the bubbles ceases. After 6 hr, 2.09 g (10 mmol) of $Br—(CH_2)_5—CO_2CH_3$ and 2.5 ml DMF are added dropwise over 10 min. The solution should be cooled to 0—5°C with an ice-bath. The mixture is then stirred at room temperature. The reaction mixture is poured into ice-water and extracted with ethyl acetate. The organic layer is washed with water and then with brine, dried over $MgSO_4$, filtered and concentrated to give a brown oil (1.6 g). After chromatography the NMR ($CDCl_3$, δ) spectrum reveals peaks at 8.66—8.43, 7.78—7.26, 8.40, 3.88, 3.68, 2.30, and 1.92—1.10.

The IR spectrum (vmax, film) reveals peaks at 3580, 3450, 3350, 3040, 1735, 1680, 1585, 1575, 1485, 1435, 1360, 1280, 1195, 1180, 1165, 815, and 715 $cm^{-1}$.

The mass spectrum reveals an ion at m/e 250.1317.

The C:H:N ratio is 62.01:7.26:10.98.

## Example 2

6-[Formyl(3-pyridinyl)amino]hexanoic acid, sodium salt (sodium salt of Example 1)

A round-bottomed flask equipped with a magnetic stirring bar is charged with 666 mg (3.0 mmol) of the ester of Example 1, 3.15 ml 1N sodium hydroxide, and 9.45 ml methanol. The mixture is stirred at room temperature under a nitrogen atmosphere for 24 hr. The solvent is removed *in vacuo* to give a yellow solid (680 mg). TLC in ethyl acetate-hexane-ethanol (10:1:1) showed only one spot at the origin.

## Example 3

6-(3-Pyridinylamino)hexanoic acid, methyl ester (Formula I: m is zero, $R_2$ is H, n + p = 5, $Y_1$ is a valence bond, and $Q_1$ is $CO_2CH_3$)

Refer to Chart B (conversion of B—3 to B—4).

A flask equipped with a magnetic stirrer is charged with 450 mg (1.8 mmol) of the product of Example 1, 4.5 ml 2N HCl and 13.5 ml MeOH. The mixture is stirred at room temperature for 18 hr. The pH of this solution is then adjusted to 5—7 and the methanol is removed under reduced pressure; diazomethane in diethyl ether-water is added and the mixture is dissolved in 200 ml of ethyl acetate. To this mixture 50 ml of saturated $NaHCO_3$ are added and equilibrated in a separatory funnel. The organic layer is washed with brine and dried over $MgSO_4$, filtered and concentrated to the crude product as an oil. This product is purified as in the preceding Examples to yield white crystals with a melting point of 60—61°C. NMR ($CDCl_3$, δ) peaks are observed at 8.04—7.78, 7.26—6.68, 3.95—3.35, 3.66, 3.14, 2.32, and 1.86—1.20.

IR (film) peaks are observed at 3396, 3382, 3039, 3022, 2947, 2928, 2866, 2853, 1724, 1590, 1577, 1519, 1474, 1462, 1652, 1645 and 414 $cm^{-1}$.

## Example 4

6-(3-Pyridinylamino)hexanoic acid, sodium salt (sodium salt of Example 3)

The product of Example 3 is dissolved in 25 ml of methanol and 8.6 ml (8.6 mmol) of 1N NaOH, is then added. The resulting cloudy suspension is stirred at room temperature for 18 hr, at which time TLC analysis confirms that the hydrolysis is complete. The methanol is removed under reduced pressure. The concentrate is diluted with water and filtered through a cotton plug. The filtrate is frozen (dry ice-acetone) and lyophilized for 48 hr to give a white granular solid.

Preparation 1

Trimethyl ortho-5-bromopentanoate

To a mixture of 74 g of 5-bromovaleronitrile (Aldrich Chemical Co.), 21 ml of dry methanol, and 250 ml of dry ether at 0°C under a nitrogen atmosphere are added with efficient stirring 40 g of hydrogen bromide over a 30-min period. The mixture is stirred for an additional 4 hr at 0°C, at which time 100 ml of dry hexane are added. The liquid is removed through a gas-dispersion tube by suction while maintaining a positive nitrogen pressure in the flask. A mixture of 200 ml of each of dry ether and dry hexane is added with stirring and the liquid is again removed by suction.

To the solid residue of imino ester hydrobromide are added 250 ml of dry ether, then 150 ml of dry methanol and 25 ml of methyl orthoformate. The mixture is stirred at room temperature for 24 hr. The mixture is cooled to about −10°C and the solution is decanted from ammonium bromide into a dry nitrogen-filled separator funnel along with about 100 ml of ether rinse. The organic solution is immediately and quickly washed with an ice-cold solution of 20 g of potassium carbonate and 300 ml of saturated saline (after the initial shaking, some water is added to dissolve precipitated solids). The organic phase is washed with saturated saline, 3 drops of pyridine are added and the solution is then dried briefly over magnesium sulfate. The solution is concentrated *in vacuo*, 150 ml of benzene are added, and the solution is again concentrated *in vacuo*. Distillation of the residue gives the product.

Example 5

3-[N-methyl-N-(4-methoxycarbonylbutyl)aminomethyl]pyridine (Formula I: m is 1, $R_2$ is $CH_3$, $n + p = 4$, $Y_1$ is a valence bond, $Q_1$ is $CO_2CH_3$)

Refer to Chart B (conversion of B—2 to B—3).

A three-neck round-bottomed flask with a magnetic stirring bar and a nitrogen inlet, is charged with 2.2 g (0.046 mol) of sodium hydride (50% dispersion in mineral oil). While purging the system with nitrogen the emulsion is washed three times with dry hexane. The remaining gray solid is suspended in 60 ml of DMF and the flask is fitted with a 25 ml equalizing pressure addition funnel. The addition funnel is charged with a solution of 5.6 g (0.046 mol) of 3-(methylaminomethyl)pyridine dissolved in 26 ml of DMF. This solution is added dropwise over 15 min to the.sodium hydride suspension under a nitrogen atmosphere to give a pale purple colored suspension. After stirring for 1 hr the suspension is treated with a solution of trimethyl-5-bromoorthopentanoate in 20 ml of DMF which is added dropwise over 30 min. The resulting mixture is stirred for 3 hrs at which time TLC indicates no remaining starting material. The reaction is quenched by the dropwise addition of water (until gas evolution ceases) and then poured into 400 ml of ether. The organic phase is washed four times with 100 ml of water, concentrated and then diluted with 50 ml of methanol. Hydrochloric acid (25 ml, 2N) is added and the resulting solution is stirred at room temperature for 15 min. The solution is neutralized by the addition of saturated aqueous sodium bicarbonate and then poured into 100 ml of saturated aqueous sodium bicarbonate. This mixture is extracted with 400 ml of ether and the organic phase is dried over $MgSO_4$, filtered, and concentrated to give 5.07 g of crude product as a yellow oil. The NMR spectrum of this material is consistent for the desired product.

Because of the low recovery (9.68 g of product expected for a 100% conversion) all aqueous washes (from the above workup) are combined and re-extracted with 400 ml of ethyl acetate. The organic phase is washed twice with water (100 ml), dried ($MgSO_4$), filtered and concentrated to give 2.17 g of a brown colored oil. The NMR spectrum indicates this material is the ortho ester of the desired product (unhydrolyzed material).

Again all aqueous washes are combined and concentrated under reduced pressure to a volume of approximately 75 ml. This is combined with the ortho ester of the desired product (unhydrolyzed material) and diluted with 100 ml of methanol and 50 ml of water. Concentrated hydrochloric acid is added dropwise until the pH is lowered to approximately 2. This solution is then stirred at room temperature for 16 hr. Solid sodium bicarbonate is added until the pH is raised to approximately 9 and methanol is removed under reduced pressure. The concentrate is diluted with 100 ml of water and extracted with 400 ml of ethyl acetate. The organic phase is washed twice with water (100 ml), dried ($MgSO_4$), filtered and concentrated to give 1.85 g of crude product as a dark brown oil. This is combined with the previously isolated 5.00 g of crude product (above) and chromatographed on 250 g of silica gel packed in chloroform-ethanol (19:1). Eluting with the packing solvent and collecting 50 ml fractions afford 2.5 g (23%) of pure title product (fractions 16—30). Fractions 31—50 contain the desired product contaminated with more polar impurities.

The NMR ($CDCl_3$, TMS, δ) spectrum reveals peaks at 8.73—7.23, 3.69, 3.51, 2.56—2.10, and 2.19.

The IR (film, vmax) reveals peaks at 2950, 2794, 1738, 1592, 1577, 1457, 1436, 1427, 1366, 1249, 1199, 1171, 1125, 1027, 861, 834, 789, and 715 $cm^{-1}$.

The mass spectrum reveals ions at m/e 236.1523, 135, and 92.

The C:H:N ratio is 65.29:8.41:11.41.

Example 6

Sodium 3-[N-methyl-N-(4-carboxybutyl)aminomethyl]pyridine (sodium salt of Example 5)

The ester of Example 5 (1.03 g, 4.36 mmol) is dissolved in 15 ml of methanol. 1N sodium hydroxide (4.35 ml, 4.35 mmol) is added and the resulting solution is stirred at room temperature for 20 hr. TLC

analysis still indicates a trace of unhydrolyzed starting material. Another 0.30 ml of 1N NaOH reagent is added and after stirring an additional 6 hr TLC analysis confirms the hydrolysis is complete. Methanol is removed under reduced pressure and the concentrate is diluted with 50 ml of water. This aqueous solution is washed with ether (organic layer discarded), filtered through a cotton plug, frozen and lyophilized to give 1.05 g of title product as a yellow granular solid.

Preparation 2

3-pyridinylmethyl-trifluoromethyl sulfonamide (triflamide)

Refer to Chart B (conversion of B—1 to B—2).

3-Pyridinylmethylamine (Aldrich Chemical Co.) is treated with trifluorosulfonic anhydride

$$[(CF_3SO_2)_2)O]$$

and triethylamine in methylene chloride at −78°C to give the title product (J. B. Hendrickson and R. Bergeron, Tetr. Lett., 3839 (1973)).

Preparation 3

3-Pyridinylmethyl-2-ethoxy-trifluoromethyl-sulfonamide (triflamide)

Refer to Chart B (conversion of B—2 to B—3 to B—6).

The title compound of Preparation 2 is alkylated with methyl bromoacetate in acetone (or glyme) using potassium carbonate as base. Using sodium hydride in DMF also gives the alkylation product. The resulting methyl ester is treated with lithium aluminium hydride in THF, for 24 hr. Saturated aqueous sodium sulfate is then added dropwise until the mixture becomes white. The solution is dried over anhydrous sodium sulfate to give the title product. The purification is carried out by either chromatography or crystallization.

Example 7

[2-(3-Pyridinylmethylamino)ethoxy]acetic acid, methyl ester (Formula I: m is 1, $R_2$ is H, n is 2, $Y_1$ is —O—, p is 1, and $Q_1$ is $CO_2CH_3$)

Refer to Chart B (conversion of B—6 to B—7 to B—8).

The title compound of Preparation 3 is alkylated using methyl bromoacetate, to give an o-alkylated triflamide (see reference cited in Preparation 2). Deprotection of triflamide using zinc in acetic acid gives the title product. Purification is carried out by either chromatography or crystallization.

Example 8

[2-(3-Pyridinylmethylamino)ethylthio]acetic acid, ethyl ester (Formula I: m is 1, $R_2$ is H, n is 2, $Y_1$ is —S—, p is 1, and $Q_1$ is $CO_2C_2H_5$)

Refer to Chart B (conversion of B—6 to B—10 to B—11 to B—12).

A round-bottomed flask equipped with a magnetic stirring bar is charged with 5.0 mmol of the title compound of Preparation 3 and 5 ml of pyridine. The mixture is cooled to 0—5°C and 1.90 g (10.0 mmol) of p-toluenesulfonyl chloride is added. The resulting mixture is allowed to stand at room temperature for 24 hr. The mixture is then cooled to 0—5°C and 0.1 ml of water is added. The mixture is allowed to warm to room temperature and stirred for 30 min. Ethyl acetate is added and the mixture is washed with water and brine, and then dried over anhydrous magnesium sulfate. Filtration and concentration yield the tosylate which is alkylated with the thio anion of ethyl 2-mercaptoacetate (Aldrich Chemical Co.), prepared with sodium hydride in DMF or potassium carbonate in glyme (or acetone), followed by chromatographic purification. The deprotection of triflamide using zinc in acetic acid, as described in Example 7, gives the title product. Purification is carried out by either chromatography or crystallization.

Example 9

[2-(3-Pyridinylmethylamino)ethylamino]acetic acid, methyl ester (Formula I: m is 1, $X_1$ is H, $Y_1$ is —NH—, n is 2, p is 1, and $Q_1$ is $CO_2CH_3$)

Refer to Chart B (conversion of B—6 to B—10 to B—11 to B—12).

The tosylate of Example 8 is alkylated according to the N-alkylation procedure of Preparation 3, to give bis-triflamide. Deprotection of triflamide according to the procedure of Example 7 gives the title product.

CHART A

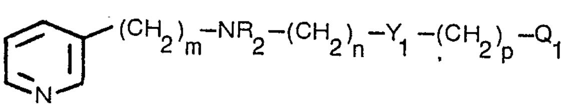

(I)

CHART B

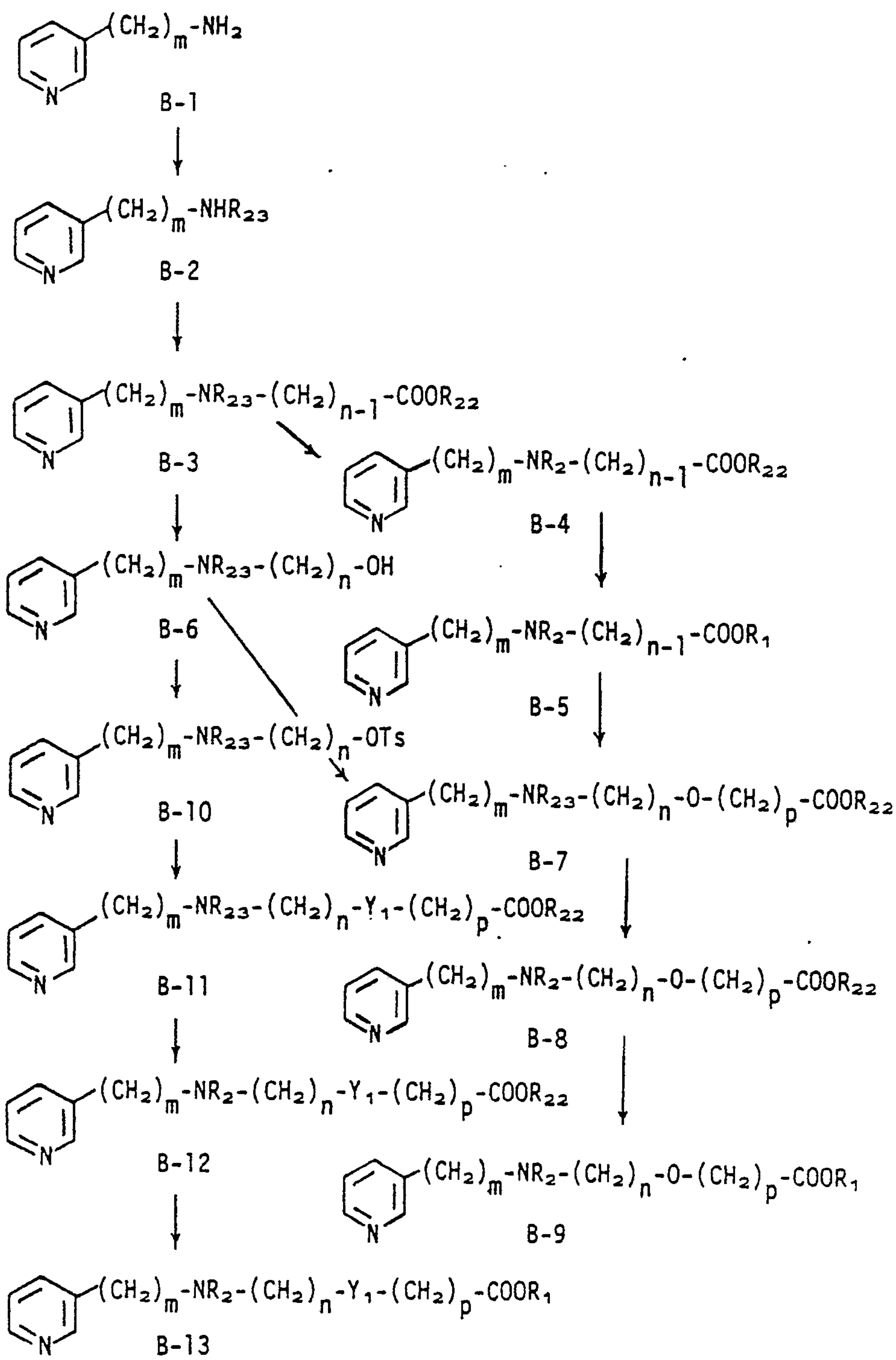

B-1

B-2

B-3

B-4

B-5

B-6

B-7

B-8

B-9

B-10

B-11

B-12

B-13

**Claims**

1. A compound of the formula

CHART A

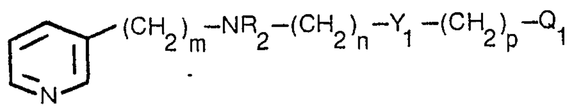

(I)

wherein

$Y_1$ is —O—, —S—, —$NR_2$— or a valence bond;

$R_2$ is H, $C_{1-5}$ alkyl or CHO;

$Q_1$ is $CH_2OH$ or $COOR_1$ wherein $R_1$ is H, a pharmacologically-acceptable cation, $C_{1-12}$ alkyl, $C_{3-10}$ cycloalkyl, $C_{7-12}$ aralkyl, phenyl or phenyl substituted by one, 2 or 3 substituents independently selected from Cl and $C_{1-3}$ alkyl;

m is zero or an integer of 1 to 6;

n is an integer of 2 to 6; and

p is zero or an integer of 1 to 6;

provided that n + p is at least 3 if $Y_1$ is a valence bond.

2. A compound of claim 1, wherein m + n + p is 2 to 7, $R_2$ is H, $Y_1$ is a valence bond, $Q_1$ is $COOR_1$ and $R_1$ is H, $CH_3$ or a pharmacologically-acceptable cation.

3. A compound of claim 1, wherein m + n + p is 2 to 7, $R_2$ is CHO, $Y_1$ is —O— or a valence bond, $Q_1$ is $COOR_1$ and $R_1$ is H, $CH_3$ or a pharmacologically-acceptable cation.

4. A compound of any preceding claim, wherein m is zero.

5. A compound of any preceding claim, wherein $Y_1$ is a valence bond.

6. A compound of claim 1, which is 6-[formyl(3-pyridinyl)amino]hexanoic acid, methyl ester or sodium salt.

7. A compound of claim 1, which is 6-(3-pyridinylamino)hexanoic acid, methyl ester or sodium salt.

8. A compound of claim 1, which is 5-[methyl(3-pyridinylmethyl)aminopentanoic acid, methyl ester or sodium salt.

9. A compound of claim 2, which is selected from [2-(3-pyridinylmethylamino)ethoxy]acetic acid, methyl ester; [2-(3-pyridinylmethylamino)ethylthio]acetic acid, ethyl ester; and [2-(3-pyridinylmethylamino)ethylamino]acetic acid, methyl ester.

**Patentansprüche**

1. Eine Verbindung der Formel

CHART A

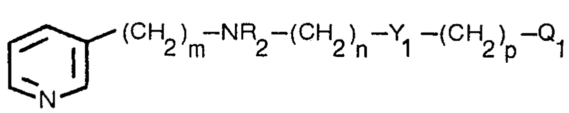

(I)

worin

$Y_1$ —O—, —S—, —$NR_2$— oder eine Valenzbindung ist;

$R_2$ H, $C_{1-5}$ Alkyl oder CHO ist;

$Q_1$ $CH_2OH$ oder $COOR_1$ ist, worin $R_1$ H, ein pharmakologisch annehmbares Kation, $C_{1-12}$ Alkyl, $C_{3-10}$ Cycloalkyl, $C_{7-12}$ Aralkyl, Phenyl oder durch einen, 2 oder 3 unabhängig aus Cl und $C_{1-3}$ Alkyl ausgewählten Substituenten substituiertes Phenyl ist;

m Null oder eine ganze Zahl von 1 bis 6 ist;

n eine ganze Zahl von 2 bis 6 ist; und

p Null oder eine ganze Zahl von 1 bis 6 ist;

vorausgesetzt, daß n + p mindestens 3 beträgt, wenn $Y_1$ eine Valenzbindung ist.

2. Eine Verbindung nach Anspruch 1, worin m + n + p 2 bis 7, $R_2$ H, $Y_1$ eine Valenzbindung, $Q_1$ $COOR_1$ und $R_1$ H, $CH_3$ oder ein pharmakologisch annehmbares Kation ist.

3. Eine Verbindung nach Anspruch 1, worin m + n + p 2 bis 7, $R_2$ CHO, $Y_1$ —O— oder eine Valenzbindung, $Q_1$ $COOR_1$ und $R_1$ H, $CH_3$ oder ein pharmakologisch annehmbares Kation ist.

4. Eine Verbindung nach einem der vorstehenden Ansprüche worin m Null ist.

5. Eine Verbindung nach einem der vorstehenden Ansprüche worin $Y_1$ eine Valenzbindung ist.

6. Eine Verbindung nach Anspruch 1, die 6-[Formyl (3-Pyridinyl)Amino]-Capronsäure, Methylester oder Natriumsalz ist.

7. Eine Verbindung nach Anspruch 1, die 6-(3-Pyridinyl-Amino)-Capronsäure, Methylester oder Natriumsalz ist.

8. Eine Verbindung nach Anspruch 1, die 5-[Methyl(3-Pyridinylmethyl)-Aminopentansäure, Methylester oder Natriumsalz ist.

9. Eine Verbindung nach Anspruch 2, ausgewählt aus der Gruppe [2-(3-Pyridinylmethylamino)äthoxy]-Essigsäure, Methylester; [2-(3-Pyridinylmethylamino)Äthylthio]-Essigsäure, Äthylester; und [2-(3-Pyridinylmethylamino)-Äthylamino]-Essigsäure, Methylester.

**Revendications**

1. Composé répondant à la formule

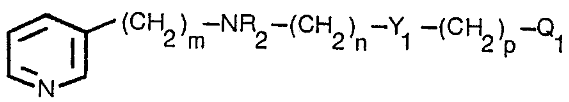

(I)

dans laquelle

$Y_1$ représente —O—, —S—, —$NR_2$— ou une liaison de valence;

$R_2$ représente H, un groupe alkyle en $C_1$ à $C_5$ ou CHO;

$Q_1$ représente un groupe $CH_2OH$ ou $COOR_1$ dans lequel $R_1$ représente H, un cation pharmacologiquement acceptable, un groupe alkyle en $C_1$ à $C_{12}$, cycloalkyle en $C_3$ à $C_{10}$, aralkyle en $C_7$ à $C_{12}$, phényle ou phényle substitué avec un, 2 ou 3 substituants choisis indépendamment entre Cl et un groupe alkyle en $C_1$ à $C_3$;

m est égal à zéro ou à un nombre entier de 1 à 6;

n est un nombre entier de 2 à 6; et

p est égal à zéro ou à un nombre entier de 1 à 6;

sous réserve que la somme n + p soit au moins égale à 3 si $Y_1$ est une liaison de valence.

2. Composé suivant la revendication 1, dans lequel la somme m + n + p est comprise dans l'intervalle de 2 à 7, $R_2$ représente H, $Y_1$ représente une liaison de valence, $Q_1$ est un groupe $COOR_1$ et $R_1$ représente H, un groupe $CH_3$ ou un cation pharmacologiquement acceptable.

3. Composé suivant la revendication 1, dans lequel la somme m + n + p est comprise dans l'intervalle de 2 à 7, $R_2$ représente un groupe CHO, $Y_1$ représente —O— ou une liaison de valence, $Q_1$ est un groupe $COOR_1$ et $R_1$ représente H, $CH_3$ ou un cation pharmacologiquement acceptable.

4. Composé suivant l'une quelconque des revendications précédentes, dans lequel m a la valeur zéro.

5. Composé suivant l'une quelconque des revendications précédentes, dans lequel $Y_1$ est une liaison de valence.

6. Composé suivant la revendication 1, qui est l'ester méthylique ou le sel de sodium de l'acide 6-[formyl(3-pyridinyl)amino]hexanoïque.

7. Composé suivant la revendication 1, qui est l'ester méthylique ou le sel de sodium de l'acide 6-(3-pyridinylamino)hexanoïque.

8. Composé suivant la revendication 1, qui est l'ester méthylique ou le sel de sodium de l'acide 5-[méthyl(3-pyridinylméthyl)aminopentanoïque.

9. Composé suivant la revendication 2, qui est choisi entre l'ester méthylique de l'acide [2-(3-pyridinylméthylamino)éthoxy]acétique; l'ester éthylique de l'acide [2-(3-pyridinylméthylamino)éthylthio]acétique; et l'ester méthylique de l'acide [2-(3-pyridinylméthylamino)éthylamino]acétique.